# EUROPEAN PATENT SPECIFICATION

(11) **EP 4 162 930 B1**
(45) Date of publication and mention of the grant of the patent: **25.09.2024**
(21) Application number: 21201925.1
(22) Date of filing: 11.10.2021
(51) Int. Cl.: A61K 9/51, A61K 9/14

(54) **PREPARATION OF DRUG-LOADED MICRO- AND NANOPARTICLES BY JET IMPINGEMENT**
HERSTELLUNG VON MIT ARZNEISTOFF BELADENEN MIKRO- UND NANOPARTIKELN DURCH STRAHLEINSCHUSS
PRÉPARATION DE NANO- ET MICROPARTICULES CHARGÉES DE MÉDICAMENT PAR PROJECTION DE JET

(43) Date of publication of application: 12.04.2023
(73) Proprietor: leon-nanodrugs GmbH, 82152 Planegg (DE)
(72) Inventor: VOGLER, Julian, 81679 Munich (DE); BRÄUER, Carsten, 81679 Munich (DE); CLÉMENT, Pascale, 81679 Munich (DE); STIENEKER, Frank, 81679 Munich (DE)
(74) Representative: Synergy IP Group AG

(56) References cited:
- EP-A2- 1 165 224
- WO-A1-2019/148147
- WO-A1-2021/046078
- TURINO L.N. ET AL.: "Nanoparticles obtained by confined impinging jet mixer: poly(lactide-co-glycolide) vs. poly-[epsilon]-caprolactone", JOURNAL DRUG DEVELOPMENT AND INDUSTRIAL PHARMACY, vol. 44, no. 6, 3 June 2018 (2018-06-03), US, pages 934 - 941, XP055895323, ISSN: 0363-9045, DOI: 10.1080/03639045.2017.1421662
- TÜRELI N.G. ET AL.: "Optimization of ciprofloxacin complex loaded PLGA nanoparticles for pulmonary treatment of cystic fibrosis infections: Design of experiments approach", INTERNATIONAL JOURNAL OF PHARMACEUTICS, vol. 515, no. 1-2, 1 December 2016 (2016-12-01), NL, pages 343 - 351, XP055895345, ISSN: 0378-5173, DOI: 10.1016/j.ijpharm.2016.10.025

## Description

### BACKGROUND

Micro- and nanoparticles formulated as drug delivery systems for active pharmaceutical ingredients play a growing role in medicine and address challenges in diagnosis, monitoring, control, prevention and treatment of diseases. The carrier material can be based on inorganic (e.g., metal nanoparticles, semi-conductor quantum dots of various sizes and shapes) or organic nanostructures, including polymers, dendrimers, micelles, liposomes, solid lipid nanoparticles and polymer-active pharmaceutical ingredient (API) conjugates.

Drug delivery systems based on poly(lactide-co-glycolic acid) (PLGA) offer multiple opportunities in terms of design and performance thanks to the various properties of PLGA that make it an ideal nanocarrier. PLGA is a biodegradable and biocompatible polymer with a wide range of degradation times that can be tuned by its molecular weight and copolymer ratio. A lower proportion of more hydrophilic glycolide residues or a higher proportion of more lipophilic lactide residues results in a longer half-life in vivo.

Although several PLGA-based systems have already been approved by both the Food and Drug Administration (FDA) and the European Medicine Agency (EMA) and are widely used in the clinics for the treatment or diagnosis of diseases, no PLGA nanomedicine formulation is currently available on the global market. One reason which hinders the industrial applicability are the challenges in scaling-up the production and the associated translation into clinical practice.

Since the particle size has an influence on the kinetics of the active ingredient release and the cellular uptake of the particles, the method of the preparation of the PLGA based micro- and nanoparticles is one of the key factors for successful marketing.

The most commonly used methods of manufacturing PLGA particles are based on emulsion processes using mechanical stirrers followed by solvent removal. However, these processes have the disadvantage that the resulting particles have a very broad particle size distribution, the processes have a low repeatability and the manufacturing conditions are sometimes harsh and therefore unsuitable for sensitive active ingredients.

Alternative microfluidic techniques were becoming more important as they allow the production of PLGA particles via a controlled process. These microfluidic techniques can be divided into droplet-based processes and continuous processes.

In the droplet-based microfluidic process, PLGA is dissolved in a water-immiscible solvent (solvent) and mixed with an aqueous solvent (non-solvent) during the process. Defined droplets are generated which, after removal of the solvent, result in defined PLGA particles. These processes are suitable for the production of microparticles, but not for the production of nanoparticles. Droplet-based methods are not suitable for high throughputs due to the low flow rates. Continuous processes in which an aqueous phase is mixed with a water-miscible solvent (such as acetone) offer alternative approaches. In continuous processes, the solvent is removed from the system, which leads to the formation of nanoparticles.

Recently, it could be shown that microfluidic technology is suitable for the preparation of PLGA-PEG nanoparticles (Mares AG, et al., 2021, PLoS ONE 16(6): e0251821. doi.org/10.1371/journal.pone.0251821). Several PLGA-PEG nanoparticles batches were formulated by manual bulk mixing and hydrodynamic flow focusing self-assembly at corresponding volumes of solvent and antisolvent. For the hydrodynamic flow focusing self-assembly microfluidic chips were used with three inlets and one outlet (microchannels of 200 µm width and 60 µm height) connected to a syringe pump with a capillary tubing. The solvent phase was injected into the central inlet and the stream width (at the outlet) was controlled by the flow rate of laterally injected antisolvent. It was demonstrated that the adjustment of the solvent and antisolvent flow rates in confined mixing area allowed to obtain broader range of diameters than with the bulk nanoprecipitation. Such microfluidic systems are known to have a high sensitivity to streamlines disturbances, microchannel clogging and fouling and solvent compatibility with chip material is sometimes a challenge.

Scalability of methods based on nanoprecipitation techniques was demonstrated by Feng 2019 (Feng et al., 2019, J Transl Med (2019) 17:200, doi.org/10.1186/s12967-019-1945-9). Three scales of mixers, including a small-scale confined impinging jet mixer, a mid-scale multi-inlet vortex mixer (MIVM) and a large-scale multi-inlet vortex mixer, were utilized in the formulation of nanoparticles comprising Lumefantrine, an anti-malaria drug and HPMCAS. Nanoparticles of 200 nm were made at all three scales of mixers by operating at equivalent Reynolds numbers (dynamic similarity) in each mixer. The disclosure does not provide information about how to conduct the method in order to obtain particles of different sizes, e.g. microparticles or nanoparticles.

As particle size and a narrow particle size distribution is always an important for both, the medicinal functioning as well as the upscaling processes in the production, the fine tuning of the method of the preparation of the PLGA based micro- and nanoparticles remains a challenge. Thus, there is still a need for robust method of production of micro- and nanoparticles that can easily be upscaled and provide a fast production of the desired product. Additionally, it is also of importance that the production process can be adapted to different needs and requirements that may emerge during the process of product development or during upscaling.

It is thus an object of the present invention to provide a method for the production of micro- and nanoparticles that is flexible in terms of adaptation to different kind of products as well as robust, fast and reliably producing the desired product. There is also a need for sufficiently controlled processes. Further objects of the invention will be clear on the basis of the following description of the invention, examples and claims.

### SUMMARY OF THE INVENTION

The present invention provides a method for preparing polymeric micro- or nanoparticles comprising a biologically active agent. The method essentially comprises the following steps:
(a) providing a jet impingement reactor comprising a reaction chamber defined by an interior surface of a chamber wall, wherein the reaction chamber has a substantially spherical spheroidal overall shape, said chamber comprising a first and a second fluid inlet, wherein the first and the second fluid inlet are arranged at opposite positions on a first central axis of the reaction chamber such as to point at one another, and wherein each of the first and the second fluid inlet comprises a nozzle; and a fluid outlet arranged at a third position, said third position being located on a second central axis of said chamber, the second central axis being perpendicular to the first central axis;
(b) providing a first stream of a first liquid composition, wherein the first liquid composition comprises an organic solution of a polymer selected from PLA, PGA, PLGA and combinations thereof, and the biologically active agent;
(c) providing a second stream of a second liquid composition, wherein the second liquid composition is an aqueous solution;
(d) simultaneously injecting the first stream at a first flow rate through the first fluid inlet and the second stream at a second flow rate through the second fluid inlet into the reaction chamber of the jet impingement reactor such as to allow the first liquid composition and the second liquid composition to mix and thereby form a third liquid composition;
(e) allowing the third liquid composition to flow out of the reaction chamber through the fluid outlet; and
(f) collecting the third liquid composition.

The jet impingement reactor is preferably designed to have nozzles that are oriented such that the first stream and the second stream are directed along the first central axis towards the centre of the reaction chamber and to allow the first stream and the second stream to collide at an angle of about 180°.

The first liquid composition which is used for the first stream that is injected into the reactor, is an organic solution of the particle-forming polymer which also comprises the biologically active ingredient, and it may be designed to be miscible or immiscible with the aqueous solution that provides the second stream. If miscible, the method can be conducted such that the mixing of the two streams in the jet impingement reactor leads to the formation of polymeric nanoparticles dispersed in a liquid phase comprising both water and organic solvent. If the first and the second liquid compositions are immiscible, the method can be controlled such that the mixing of the two streams in the jet impingement reactor leads to the formation of an emulsion containing dispersed organic droplets from which polymeric microparticles can be obtained in a subsequent step. In either case, the biologically active ingredient will be incorporated within the polymeric particles.

### DESCRIPTION OF THE DRAWING

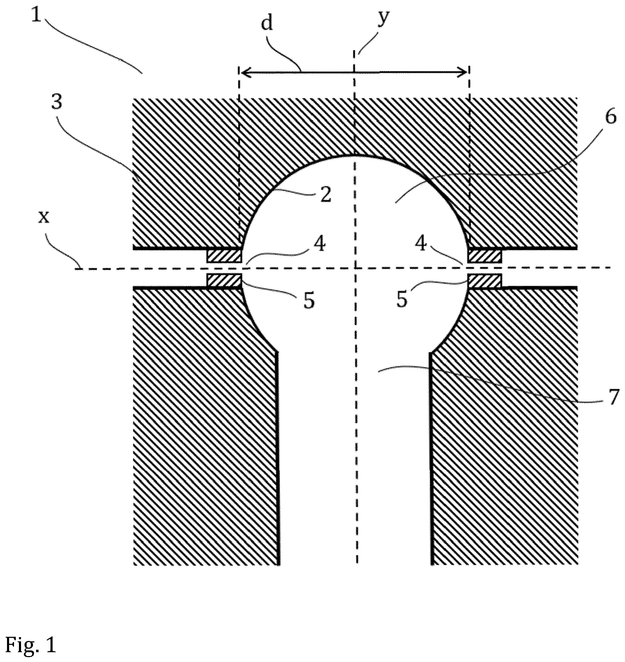

Figure 1, which is not to scale, depicts an example of a jet impingement reactor (1) that is useful for carrying out the method of the invention. The reaction chamber (6) defined by the interior surface (2) of the chamber wall (3) is substantially spherical, except for the two fluid inlets (4) and the fluid outlet (7). The fluid inlets (4) are arranged at opposite positions on a first central axis (x) of the reaction chamber (6) and point at one another. Each of the fluid inlets (4) comprises a nozzle (5), which is a plain orifice nozzle in this embodiment. The fluid outlet (7) is positioned on a second central axis (y) which is perpendicular to the first central axis (x). The distance (d) between the two nozzles (4) is substantially the same as the diameter of the spherical reaction chamber (6).

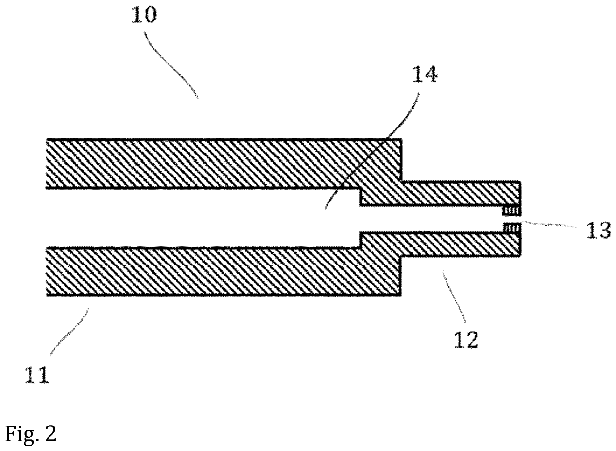

Figure 2 depicts a fluid inlet connector (10) according to one embodiment of the invention. The connector (10) has an upstream end (11), a downstream end (12) holding a nozzle (13) at a downstream position of the downstream end (12), and a fluid conduit (14) for conducting a fluid from the upstream end (11) to the downstream end (12). The fluid inlet connector (10) is designed to provide a fluid inlet for a jet impingement reactor (not shown) according to the invention, and to be reversibly insertable into the wall such of such reactor. The figure is not to scale.

### DETAILED DESCRIPTION OF THE INVENTION

In a first aspect, the present invention provides a method for preparing polymeric micro- or nanoparticles comprising a biologically active agent. The method comprises steps (a) to (f), wherein the steps are defined as follows:
(a) providing a jet impingement reactor comprising a reaction chamber defined by an interior surface of a chamber wall, wherein the reaction chamber has a substantially spheroidal overall shape, said chamber comprising a first and a second fluid inlet, wherein the first and the second fluid inlet are arranged at opposite positions on a first central axis of the reaction chamber such as to point at one another, and wherein each of the first and the second fluid inlet comprises a nozzle; and a fluid outlet arranged at a third position, said third position being located on a second central axis of said chamber, the second central axis being perpendicular to the first central axis;
(b) providing a first stream of a first liquid composition, wherein the first liquid composition comprises an organic solution of a polymer selected from PLA, PGA, PLGA and combinations thereof, and the biologically active agent;
(c) providing a second stream of a second liquid composition, wherein the second liquid composition is an aqueous solution;
(d) simultaneously injecting the first stream at a first flow rate through the first fluid inlet and the second stream at a second flow rate through the second fluid inlet into the reaction chamber of the jet impingement reactor such as to allow the first liquid composition and the second liquid composition to mix and thereby form a third liquid composition;
(e) allowing the third liquid composition to flow out of the reaction chamber through the fluid outlet; and
(f) collecting the third liquid composition.

In the context of the invention, polymeric microparticles are particles based on a polymeric carrier having an average particle size in the range of about 1 µm to about 500 µm, whereas polymeric nanoparticles have an average particle size in the range from about 10 nm to about 1 µm. The average particle size is preferably the z-average determined by dynamic light scattering or laser diffraction. Micro- and nanoparticles may have any type of morphology. For example, a microcapsule a specific type of microparticle having a core and a shell, wherein the core and the shell show differences in their structure or composition. A biologically active agent, in this context, is a compound or material which, when administered to a subject, has a biological effect based on which the agent may be useful as an active pharmaceutical ingredient or diagnostic agent. A biologically active agent may also be referred to as active agent, active ingredient or the like. A drug substance is an example of a biologically active agent.

PLA means poly(lactic acid), also referred to polylactide; PLG means poly(glycolic acid), or polyglycolide; and PLGA is used to refer to the copolymer poly(lactic-co-glycolic acid) or polylactide-co-glycolide.

An organic solution of a polymer, as used herein, refers to a liquid composition in which the polymer exists in the dissolved state, and wherein the liquid constituent(s) are predominantly organic. An aqueous solution is a liquid composition having a single phase wherein the sole or the major liquid constituent is water.

A flow rate, in the context of the invention, means the rate at which a defined volume of a stream flows in the direction of flow, and is therefore expressed as a volume per time unit, such as mL/min.

The inventors have unexpectedly found that jet impingement technology using a reactor as described herein may be employed to produce both polymeric micro- and nanoparticles, wherein either type of particles is loaded with an active agent.

Moreover, the type of particles that are obtained may be controlled by the selection of the ingredients in particular of the first liquid composition and to some extent by the process parameters, in particular the flow rates.

A particularly useful type of jet impingement reactor is one whose reaction chamber has a substantially spheroidal overall shape. Such reactor is described, for example, in the co-pending patent application no. EP21192535.9.

As used herein, a substantially spheroidal overall shape means that at least the larger part of reaction chamber as defined by the internal surface of the chamber wall has the shape of a sphere or is similar to a sphere. For example, the spheroid may be shaped such that some of its cross sections are ellipses. In one preferred embodiment, all parts or portions of the reaction chamber or of the interior surface of the chamber wall except for those portions that hold or define an inlet or an outlet opening are substantially spheroidal, or even spherical.

Figure 1 illustrates an example of a jet impingement reactor that is useful for carrying out the invention.

In case the outlet, which is typical relatively large in diameter compared to the diameter of the nozzles or inlet openings, is understood as a deviation from the otherwise spherical shape of the reaction chamber, the shape of the reaction chamber may also be described as a spherical cap, also referred to as a spherical dome. In a preferred embodiment, such spherical cap has a height, a basis, and a radius along the first central axis (i.e. on which the two fluid inlets are positioned), wherein the height is larger than said radius, and wherein the basis is defined by the fluid outlet. In other words, the spherical dome formed by the reaction chamber is larger in volume than a corresponding hemisphere, which also means that diameter of the outlet opening is smaller than the largest diameter of the reaction chamber. In a specific embodiment, the height of the dome is in the range of about 110% to about 170% of the radius. For example, the height may be about 120% to about 160% of the radius, such as about 120%, about 130%, about 140%, about 150%, or about 160% of the radius.

Another preferred feature of the reactor relates to the arrangement of the nozzles. As mentioned, each of the first and the second fluid inlet comprises a nozzle, and in the assembled state of the reactor, the distance between the nozzle of the first fluid inlet and the nozzle of the second fluid inlet is the same or smaller than the diameter of the reaction chamber along the first central axis. This is in contrast to some jet reactors known in the art which have nozzles that are retracted. In a preferred embodiment of the invention, the nozzles - more precisely their downstream ends - are neither retracted nor do they protrude into the reaction chamber, but they are substantially aligned with the interior surface of the reaction chamber wall.

In a further preferred embodiment, the jet impingement reactor is characterised in that the nozzles of the first and second fluid inlet are arranged such as to direct a first and a second fluid stream along the first central axis towards the centre of the chamber and to allow the first stream and the second stream to collide at an angle of about 180°. As used herein, the collision at an angle of about 180° may also be referred to as a frontal collision. In this context, the expression "about" means that the actual angle is sufficiently close to 180° to ensure that the collision of the first stream and the second stream results in a rapid and highly turbulent fluid flow in the mixing zone, such that thorough mixing takes place within an extremely short time, e.g., typically within a matter of milliseconds.

In a further particularly preferred embodiment, the reaction chamber is free of other inlets or outlets, i.e. of other openings than those described herein as inlets or outlet. In other words, the first and the second inlet and the outlet represent the only openings of the reaction chamber that are provided in the chamber wall. This is also in contrast to some known jet impingement reactors which exhibit one or more additional inlets, such as an inlet for a gas to be introduced to the reaction chamber or an outlet for degassing purposes. However, as the inventors have found, such additional inlets or outlets may also negatively interfere with the impingement process and result in uncontrolled precipitation or the building up of contamination in such additional openings, and that a reactor as described herein brings about the advantage of better control over the interaction and mixing of the first stream with the second stream, improved cleanability and increased batch-to-batch consistency.

In a further preferred embodiment, the jet impingement reactor is characterised in that the nozzle of the first inlet has a first orifice diameter and the nozzle of the second fluid inlet has a second orifice diameter, and in that the first orifice diameter and/or the second orifice diameter are in the range of 20 µm to 500 µm. Preferably, both the first orifice diameter and the second orifice diameter are in the range of 20 µm to 500 µm, or in the range of about 50 µm to 500 µm. Also preferred are reactor configurations in which at least one of the orifice diameters is about 500 µm, about 400 µm, about 300 µm, about 200 µm, about 100 µm, about 50 µm, or about 20 µm, respectively. Even smaller diameters, e.g. below 20 µm, may be considered.

In one specific embodiment, the diameters of the first and the second nozzle (i.e. nozzle orifice) are the same, such as about 300 µm, about 200 µm, about 100 µm. The inventors have found however that even better results may be obtained with two nozzle diameters that differ from one another. It is therefore a preferred embodiment that the reactor used according to the invention has two nozzles that differ in size. In other words, the first orifice diameter is larger than the second orifice diameter, according to this preference. Such asymmetric nozzle configuration may have further advantages: For example, it may be used to minimise the introduction of a solvent that is required for processing purposes. It may also be used for the generation of two liquid streams that have different flow rates but similar kinetic energy as they are injected through the nozzles into the reaction chamber where they collide.

In one embodiment, the diameter of the first nozzle (i.e. its orifice) is at least 20% larger than that of the second nozzle. In a further embodiment, the ratio of the first orifice diameter to the second orifice diameter is from about 1.2 to about 5. For example, the following nozzle pairs may be used, wherein the first value represents the approximate diameter of the first orifice and the second value the approximate diameter of the second orifice: 100 µm and 50 µm; 200 µm and 100 µm; 200 µm and 50 µm; 300 µm and 200 µm; 300 µm and 100 µm; 300 µm and 50 µm; 400 µm and 300 µm; 400 µm and 200 µm; 400 µm and 100 µm; 400 µm and 50 µm; 500 µm and 400 µm; 500 µm and 300 µm; 500 µm and 200 µm; 500 µm and 100 µm; 500 µm and 50 µm.

In a further embodiment, the jet impingement reactor used for carrying out the method of the invention is characterised in that each of the first and the second inlet is provided by a fluid inlet connector having an upstream end, a downstream end holding the nozzle of the first or second fluid inlet, and a fluid conduit for conducting a fluid from the upstream end to the downstream end. Furthermore, the downstream end of each fluid inlet connector is reversibly insertable into the chamber wall such as to provide the first and the second fluid inlet. Optionally, the fluid inlet connector that provides the first and/or the second fluid inlet is optionally affixed to the chamber wall by means of a single ferrule fitting or a double ferrule fitting.

Further optional features and preferences relating to jet impingement reactors that are useful for carrying out the present invention are disclosed in patent application no. EP21192535.9.

As mentioned, the polymer on which the particles are based and which is introduced via the first liquid composition or the first stream, respectively, is selected from PLA, PGA, PLGA and combinations thereof. As generally known to the skilled person, various potentially suitable types and grades of these polymers are available. They may differ in their biodegradation kinetics and thus in the drug release profiles that may be achievable with them. The polymer selection will therefore also depend on the specific product application for which the method is to be used. In general, the pharmaceutical grades of FDA-approved PLA, PGA and PLGA are preferred.

In one embodiment, the polymer comprised in the first liquid composition comprises a PLGA. In a further embodiment, the PLGA has a lactide:glycolide ratio of 75:25, an acidic end group, and a mean molecular weight of approximately 20 kDa. Such PLGA may also be combined with another PLGA polymer, or with a PLA or PGA.

The inventors have found that the method works particularly well when the first liquid composition, i.e. the organic solution of the polymer that also comprises the active agent, exhibits a moderate viscosity. The viscosity is substantially affected by the polymer concentration of the organic solution, but also to some extent by the polymer type and grade, the organic solvent(s) and the type and amount of active agent. While it would in principle be desirable to use a high polymer concentration in order to minimise the required amounts of organic solvent, the inventors have found that the method works much more efficiently if the viscosity of the organic solution is not too high. In one of the preferred embodiments, the first liquid composition is selected such that it has a viscosity of not more than 40 mPas*s. In other embodiments, the ingredients and their amounts are selected such that the viscosity of the first liquid composition is in the range from about 5 mPas*s to about 40 mPas*s, or from about 10 mPas*s to about 35 mPas*s, respectively. As used herein, the viscosity is the dynamic measured at or close to normal conditions, i.e. normal temperature and normal pressure.

With respect to the second liquid composition representing an aqueous solution which is used for the second stream to be injected into the reactor, the inventors have found that it is highly desirable that it comprises a stabiliser capable of physically stabilising the polymeric particles as they are formed. In particular when using the method of the invention to produce nanoparticles, it is useful to stabilise these against agglomeration. Potentially suitable stabilisers for this purpose are generally known to a person skilled in the art. They include surfactants, such as ionic (i.e. cationic, anionic, zwitterionic, or polyelectrolyte surfactants) or non-ionic surfactants, and amphiphilic or hydrophilic polymers, in particular water-soluble polymers.

In one of the preferred embodiments, the second liquid composition comprises a surfactant. In a further embodiment, the surfactant is a non-ionic surfactant. According to a further preferred embodiment, this non-ionic surfactant is selected from the group of polysorbates and poloxamers. Also preferred are polysorbate and poloxamer grades that are approved as excipients in drug products for parenteral use, for example polysorbate 20, polysorbate 80, or poloxamer 144. In a specific embodiment, the second liquid composition is an aqueous solution of polysorbate 20, wherein the concentration of polysorbate 20 in the second liquid composition is in the range from about 0.1 wt.% to 2 wt.%. Optionally, the polysorbate 20 may also be lower, such as from about 0.01 wt.% to about 0.1 wt.%; or it may be about 0.2 wt.%, about 0.5 wt.%, or about 1 wt.%, respectively.

As mentioned, the inventors have found that polymer micro- and nanoparticles are advantageously prepared using a jet impingement reactor having an asymmetric configuration with respect to the nozzle diameters in that the diameter of the first nozzle is smaller than the diameter of the second nozzle. It has also been found that it is advantageous to use a higher flow rate for the second stream than for the first stream.

Accordingly, it is a further preferred embodiment which is characterised in that the second flow rate is larger than the first flow rate. For example, the second flow rate may be at least 10% higher than the first flow rate. Preferably, the second flow rate is at least about 20% larger (i.e. higher) than the first flow rate. In this context, the basis of the percentages is the first flow rate, i.e. the lower of the two flow rates.

In a further preferred embodiment, the ratio of the first flow rate to the second flow rate is preferably in the range from 1.5 : 1 to 20 : 1, and in particular from 2 : 1 to 10 : 1. Another useful flow rate ratio ranges from about 2 : 1 to 5 : 1.

With respect to the total flow rate of the two liquid streams injected into the reactor, which is the sum of the first flow rate and the second flow rate, this is preferably selected in the range from about 1 ml/min to about 500 ml/min when performing the method of the invention. Also preferred are total flow rates of at least about 10 ml/min. In a further preferred embodiment, the total flow rate is at least about 50 ml/min. It was found by the inventors that these flow rates, in particular in combination with other preferences described herein, are advantageous for the preparation of polymeric particles based on the jet impingement technology according to the invention.

As mentioned, the polymeric micro- and nanoparticles thus prepared comprise the biologically active ingredient. Depending on the particle morphology, the biologically active ingredient may be incorporated within the particles in the form of an embedded or encapsulated material, or it may be associated with the particles (e.g. by surface adsorption). In a preferred embodiment, the biologically active agent is embedded and/or encapsulated within the polymeric micro- or nanoparticles.

As described above, the method of the invention can be conducted such as to obtain polymeric microparticles or it may be conducted such as to produce polymeric nanoparticles. The inventors have found that a particularly advantageous manner of controlling the process and the resulting particle type is by specifically designing the first liquid composition, i.e. the organic polymer solution that also contains the active agent, to be either miscible or immiscible with the aqueous solution (e.g. the aqueous surfactant solution) that represents the second liquid composition, which is achieved primarily by selecting either a water-miscible or water-immiscible organic solvent (or solvent mixture) as the sole or primary liquid constituent of the first liquid composition. In general, the use of a first liquid composition which is miscible with the second liquid composition is particularly advantageous for preparing polymeric nanoparticles, and the use of a first liquid composition which is immiscible with the second liquid composition is particularly advantageous for preparing polymeric microparticles.

Accordingly, in one of the preferred embodiments or group of embodiments, the first liquid composition is miscible with the second liquid composition. This may be achieved by selecting a suitable organic solvent which is miscible with water. For example, the organic solvent comprised in the first liquid composition may be selected from acetone, DMSO, methanol, acetonitrile, or any combinations thereof. A further organic solvent - even a solvent that is as such not miscible with water - may also be present as long as the resulting liquid composition is miscible with the second liquid composition.

In one specific embodiment, the first liquid composition comprises acetone. In a further embodiment, the first liquid composition is a solution of PLGA in acetone. If a solution of PLGA in acetone is used as the first liquid composition, it is also preferred that the polymer concentration is not higher than about 320 mg/ml and/or that the viscosity of the solution is not higher than about 40 mPa*s. With respect to alternative polymers or specific types or grades of PLGA that may be used, reference is made to the respective discussion herein-above.

As mentioned, selecting a first liquid composition which is miscible with the second liquid composition is particularly advantageous for preparing polymeric nanoparticles. The inventors found that these nanoparticles form rather rapidly such that they are present in the third liquid composition that is formed by mixing the first and the second stream in the jet impingement reactor. As described before, these nanoparticles also comprise the biologically active agent; preferably, the active agent is embedded and/or encapsulated within the polymeric nanoparticles comprised in the third liquid composition.

In an alternative but also preferred embodiment or group of embodiments, the first liquid composition is immiscible with the second liquid composition. Again, this is achieved primarily by the appropriate choice of the sole or major organic solvent used in the first liquid composition. An organic solvent that is water-immiscible should in this case be selected. For example, the first liquid composition may comprise an organic solvent that is selected from ethyl acetate and/or dichloromethane. In one of the preferred embodiments within this group of embodiments characterised in that the first liquid composition is immiscible with the second liquid composition, first liquid composition is a solution of PLGA in dichloromethane.

If a solution of PLGA in dichloromethane is used as the first liquid composition, it is further preferred that its polymer concentration is not higher than about 620 mg/ml and/or that its viscosity is not higher than 40 mPa*s.

Particularly preferred when using a first liquid composition that is immiscible with the second liquid composition is the use of a stabiliser such as a surfactant in the second liquid composition. This is also because in this case the simultaneous injection of the first stream and the second stream into the reaction chamber of the jet impingement reactor according to step (d) of the method of the invention will lead to the formation of an emulsion, which is the form in which the third liquid composition is obtained. Inherently, an emulsion needs to be physically stabilised to prevent coalescence.

The emulsion that is obtained as the third liquid composition is preferably one having a continuous aqueous phase and a dispersed organic phase. In other words, it is an emulsion of the type o/w. The formation of this type of emulsion is supported by the appropriate selection of the respective compositions from which the emulsion is prepared, and by the relative amounts of these compositions that are injected into the reactor. Following the guidance and the preferences provided above with respect to the first and the second flow rate, the skilled person would generally know which selections would lead to the formation of an o/w-emulsion.

In order to obtain the polymeric microparticles, a further step is required, which is the removal of the organic solvent. It is therefore a further preferred embodiment according to which the method of the invention comprises a further step (g) in which the organic solvent is removed from the third liquid composition such as to obtain polymeric microparticles. In this context, the organic solvent should be understood as the sole organic solvent or the organic solvent combination used in the second liquid composition, as the case may be. Moreover, it may not be necessary to remove the entire amount of organic solvent present in the third liquid composition as long as a substantial portion or fraction of the solvent is removed, to the effect that the polymer can no longer be dissolved in the dispersed phase of the o/w-emulsion.

Any suitable method for removing the organic solvent or solvents from the emulsion may be used for step (g), including solvent exchange, solvent extraction, solvent evaporation, or any combination of these. For example, solvent exchange and solvent extraction may be achieved by dialysis or cross-flow filtration of the emulsion against an aqueous phase. Solvent evaporation may be achieved by applying heat and/or vacuum to the emulsion. Optionally, the emulsion may be dried entirely, i.e. freeze dried, which will not only remove the organic solvent but also the water contained in the emulsion or the third liquid composition, respectively. The drying may also be performed such that microparticles are obtained which may be redispersed by combining the dried composition with an aqueous carrier.

Further optional features and preferences are derivable from the following examples and the patent claims.

### EXAMPLES

### Example 1

PLGA nanoparticles comprising risperidone (Risp) as biologically active agent were prepared according to the invention. In this series of experiments, the first liquid composition was an organic solution of risperidone and PLGA in acetone having a risperidone concentration of 6 mg/ml, and the second liquid composition was a solution of polysorbate 20 in demineralised water. The PLGA had a lactide:glycolide ratio of 75:25, an acidic end group, and an average molecular weight of approximately 20 kDa. The total flow rate was 55 ml/min, and the ratio of the second flow rate to the first flow rate was 4 : 1. The parameters that were varied were the PLGA concentration in the first liquid composition and the polysorbate 20 (PS20) concentration in the second liquid composition.

A jet impingement reactor according to EP1165224 was used at room temperature to carry out the mixing process. The nozzle of the first fluid inlet through which the first liquid composition was injected into the reaction chamber had a pinhole diameter of 100 µm, that of the second fluid inlet had a diameter of 200 µm.

The resulting mixture comprised risperidone nanoparticles, whose average size and polydispersity were evaluated. Subsequently, the acetone was removed at 42 °C and a vacuum of 800 mbar, and the particles were again characterised with respect to their average particle size (z-average) and polydispersity index (PDI). In addition, the release of risperidone of one of the series was measured by dialysis against PBS pH 7.4 at 45 °C using a standard dialysis cassette with a 10 kDa MWCO cellulose membrane. Risperidone was quantified in samples withdrawn from the dialysis cassette by HPLC.

The results of the experiments are provided in table 1.

**Table 1**

| | Series 1A | Series 1B | Series 1C | Series 1D |
|---|---|---|---|---|
| PLGA conc. | 12 mg/ml | 18 mg/ml | 30 mg/ml | 54 mg/ml |
| PS20 conc. | 0.2 wt.% | 0.3 wt.% | 0.6 wt.% | 1.0 wt.% |
| z-average | 789 nm | 465 nm | 1,959 nm | 106 nm |
| PDI | 0.28 | 0.27 | 0.26 | 0.25 |
| z-average* | 181 nm | 176 nm | 34 nm | 81 nm |
| PDI* | 0.27 | 0.31 | 0.25 | 0.19 |
| Risp release | nd | nd | nd | 80% at 2 h |

| | | | | |
|---|---|---|---|---|
| * after solvent removal | | | | |

### Example 2

In this series of experiments, placebo microparticles were prepared. The first liquid composition was an organic solution of PLGA in dichloromethane, and the second liquid composition was a solution of polysorbate 20 (PS20) or polyvinyl alcohol (PVA) (both 0.5 wt.%) in demineralised water. The same PLGA as in example 1 was used. The total flow rate (TFR) was 150 ml/min. The flow rate ratio (FRR) between the second flow rate (aqueous surfactant solution) and the first flow rate (organic PLGA solution) and also the PLGA concentration in the organic solution were varied.

Again, a jet impingement reactor according to EP1165224 was used at room temperature to carry out the mixing process. Pinhole diameters of 200 µm or 300 µm were used for the organic stream and 300 µm for the aqueous stream, respectively (see table 2). The resulting mixture already comprised microparticles, whose average particle size was evaluated. Subsequently, the organic solvent was removed at room temperature and a vacuum of 800 mbar, and the particles were again characterised with respect to their average particle size (z-average).

The results are provided in table 2.

**Table 2**

| | Series 2A | Series 2B | Series 2C | Series 2D | Series 2E |
|---|---|---|---|---|---|
| Pinholes* | 200/300µm | 200/300µm | 300/300µm | 300/300µm | 300/300µm |
| PLGA conc. | 300 mg/ml | 300 mg/ml | 50 mg/ml | 50 mg/ml | 50 mg/ml |
| Surfactant | PS20 | PS20 | PS20 | PVA | PVA |
| FRR | 2:1 | 4:1 | 2:1 | 2:1 | 4:1 |
| z-average | 8.1 µm | 7.9 µm | 14.5 µm | 3.6 µm | 3.4 µm |
| z-average** | 7.9 µm | 11.0 µm | 5.2 µm | 3.6 µm | 2.6 µm |

| | | | | | |
|---|---|---|---|---|---|
| * first fluid inlet / second fluid inlet ** after solvent removal | | | | | |

## Claims

1. A method for preparing polymeric micro- or nanoparticles comprising a biologically active agent, the method comprising the steps of:
(a) providing a jet impingement reactor comprising a reaction chamber defined by an interior surface of a chamber wall, wherein the reaction chamber has a substantially spheroidal overall shape, said chamber comprising
- a first and a second fluid inlet, wherein the first and the second fluid inlet are arranged at opposite positions on a first central axis of the reaction chamber such as to point at one another, and wherein each of the first and the second fluid inlet comprises a nozzle; and
- a fluid outlet arranged at a third position, said third position being located on a second central axis of said chamber, the second central axis being perpendicular to the first central axis;
(b) providing a first stream of a first liquid composition, wherein the first liquid composition comprises an organic solution of
- a polymer selected from PLA, PGA, PLGA and combinations thereof; and
- the biologically active agent;
(c) providing a second stream of a second liquid composition, wherein the second liquid composition is an aqueous solution;
(d) simultaneously injecting the first stream at a first flow rate through the first fluid inlet and the second stream at a second flow rate through the second fluid inlet into the reaction chamber of the jet impingement reactor such as to allow the first liquid composition and the second liquid composition to mix and thereby form a third liquid composition;
(e) allowing the third liquid composition to flow out of the reaction chamber through the fluid outlet; and
(f) collecting the third liquid composition.

2. The method of claim 1, wherein the distance between the nozzle of the first fluid inlet and the nozzle of the second fluid inlet is the same or smaller than the diameter of the reaction chamber along the first central axis.

3. The method of claim 1 or 2, wherein the nozzles are arranged such as to direct the first stream and the second stream along the first central axis towards the centre of the reaction chamber and to allow the first stream and the second stream to collide at an angle of about 180°.

4. The method of any one of the preceding claims, wherein the reaction chamber is free of other inlets or outlets.

5. The method of any one of the preceding claims, wherein the nozzle of the first inlet has a first orifice diameter and the nozzle of the second inlet has a second orifice diameter, wherein the first orifice diameter and/or the second orifice diameter is in the range of 20 µm to 500 µm, and wherein first orifice diameter is optionally larger than the second orifice diameter, the ratio of the first orifice diameter to the second orifice diameter optionally being from 1.2 to 5.

6. The method of any one of the preceding claims, wherein the polymer comprised in the first liquid composition comprises PLGA, and wherein the PLGA optionally has a lactide:glycolide ratio of 75:25, an acidic end group, and a mean molecular weight of approximately 20 kDa.

7. The method of any one of the preceding claims, wherein the first liquid composition has a viscosity of not more than 40 mPas*s.

8. The method of any one of the preceding claims, wherein the second liquid composition comprises a surfactant,wherein the surfactant is optionally a non-ionic surfactant selected from polysorbates such as polysorbate 20 and poloxamers.

9. The method of any one of the preceding claims, wherein the second flow rate is larger than the first flow rate, and wherein the ratio of the first flow rate to the second flow rate is preferably in the range from 1.5 : 1 to 20 : 1, and in particular from 2 : 1 to 10 : 1, and wherein the sum of the first flow rate and the second flow rate is preferably at least 50 ml/min.

10. The method of any one of the preceding claims, wherein the first liquid composition is miscible with the second liquid composition.

11. The method of claim 10, wherein the first liquid composition comprises an organic solvent selected from acetone, DMSO, methanol, acetonitrile, or any combinations thereof, and wherein the first liquid composition is optionally a solution of PLGA in acetone.

12. The method of claim 10 or 11, wherein the third liquid composition comprises polymeric nanoparticles.

13. The method of any one of claims 1 to 9, wherein the first liquid composition is immiscible with the second liquid composition.

14. The method of claim 13, wherein the first liquid composition comprises an organic solvent selected from ethyl acetate and/or dichloromethane, and wherein the first liquid composition is preferably a solution of PLGA in dichloromethane.

15. The method of claim 13 or 14, further comprising a step of
(g) removing the organic solvent from the third liquid compositions such as to obtain polymeric microparticles, and wherein the organic solvent is preferably removed by solvent exchange, solvent extraction and/or solvent evaporation.

## Patentansprüche

1. Verfahren zur Herstellung von polymeren Mikro- oder Nanopartikeln, umfassend ein biologisch aktives Mittel, wobei das Verfahren die Schritte umfasst:
(a) Bereitstellen eines Strahleinschussreaktors, umfassend eine Reaktionskammer, die durch eine Innenoberfläche einer Kammerwand definiert ist, wobei die Reaktionskammer eine im Wesentlichen kugelige Gesamtform hat, wobei die Kammer umfasst:
- einen ersten und einen zweiten Fluideinlass, wobei der erste und der zweite Fluideinlass in gegenüber liegenden Positionen auf einer ersten Zentralachse der Reaktionskammer so angeordnet sind, dass sie zueinander weisen, und wobei jeder von dem ersten und dem zweiten Fluideinlass eine Düse umfasst; und
- einen Fluidauslass, der an einer dritten Position angeordnet ist, wobei die dritte Position sich auf einer zweiten Zentralachse der Kammer befindet, wobei die zweite Zentralachse rechtwinklig zu der ersten Zentralachse ist;
(b) Bereitstellen eines ersten Stroms einer ersten flüssigen Zusammensetzung, wobei die erste flüssige Zusammensetzung eine organische Lösung von
- einem Polymer ausgewählt aus PLA, PGA, PLGA und Kombinationen davon; und
- dem biologisch aktiven Mittel umfasst;
(c) Bereitstellen eines zweiten Stroms einer zweiten flüssigen Zusammensetzung, wobei die zweite flüssige Zusammensetzung eine wässrige Lösung ist;
(d) simultanes Injizieren des ersten Stroms mit einer ersten Flussrate durch den ersten Fluideinlass und des zweiten Stroms mit einer zweiten Flussrate durch den zweiten Fluideinlass in die Reaktionskammer des Strahleinschussreaktors, um so zu ermöglichen, dass sich die erste flüssige Zusammensetzung und die zweite flüssige Zusammensetzung mischen und dadurch eine dritte flüssige Zusammensetzung bilden;
(e) Zulassen, dass die dritte flüssige Zusammensetzung durch den Fluidauslass aus der Reaktionskammer herausfließt; und
(f) Auffangen der dritten flüssigen Zusammensetzung.

2. Verfahren nach Anspruch 1, wobei der Abstand zwischen der Düse des ersten Fluideinlasses und der Düse des zweiten Fluideinlasses der gleiche wie der Durchmesser der Reaktionskammer entlang der ersten Zentralachse oder kleiner als dieser ist.

3. Verfahren nach Anspruch 1 oder 2, wobei die Düsen so angeordnet sind, dass sie den ersten Strom und den zweiten Strom entlang der ersten Zentralachse in Richtung des Zentrums der Reaktionskammer führen und ermöglichen, dass der erste Strom und der zweite Strom mit einem Winkel von etwa 180° kollidieren.

4. Verfahren nach einem der vorhergehenden Ansprüche, wobei die Reaktionskammer frei von anderen Einlässen oder Auslässen ist.

5. Verfahren nach einem der vorhergehenden Ansprüche, wobei die Düse des ersten Einlasses einen ersten Öffnungsdurchmesser hat, und die Düse des zweiten Einlasses einen zweiten Öffnungsdurchmesser hat, wobei der erste Öffnungsdurchmesser und/oder der zweite Öffnungsdurchmesser im Bereich von 20 µm bis 500 µm liegt/liegen, und wobei der erste Öffnungsdurchmesser gegebenenfalls größer als der zweite Öffnungsdurchmesser ist, wobei das Verhältnis des ersten Öffnungsdurchmessers zu dem zweiten Öffnungsdurchmesser gegebenenfalls 1,2 bis 5 beträgt.

6. Verfahren nach einem der vorhergehenden Ansprüche, wobei das in der ersten flüssigen Zusammensetzung enthaltene Polymer PLGA umfasst, und wobei das PLGA gegebenenfalls ein Lactid:Glycolid-Verhältnis von 75:25, eine saure Endgruppe und ein mittleres Molekulargewicht von annähernd 20 kDa hat.

7. Verfahren nach einem der vorhergehenden Ansprüche, wobei die erste flüssige Zusammensetzung eine Viskosität von nicht mehr als 40 mPas s hat.

8. Verfahren nach einem der vorhergehenden Ansprüche, wobei die zweite flüssige Zusammensetzung ein Tensid umfasst, wobei das Tensid gegebenenfalls ein nicht-ionisches Tensid ausgewählt aus Polysorbaten, wie Polysorbat 20, und Poloxameren ist.

9. Verfahren nach einem der vorhergehenden Ansprüche, wobei die zweite Flussrate größer als die erste Flussrate ist, und wobei das Verhältnis der ersten Flussrate zu der zweiten Flussrate vorzugsweise im Bereich von 1,5:1 bis 20:1 und insbesondere 2:1 bis 10:1 liegt, und wobei die Summe der ersten Flussrate und der zweiten Flussrate vorzugsweise mindestens 50 ml/min beträgt.

10. Verfahren nach einem der vorhergehenden Ansprüche, wobei die erste flüssige Zusammensetzung mit der zweiten flüssigen Zusammensetzung mischbar ist.

11. Verfahren nach Anspruch 10, wobei die erste flüssige Zusammensetzung ein organisches Lösungsmittel ausgewählt aus Aceton, DMSO, Methanol, Acetonitril oder beliebigen Kombinationen davon umfasst, und wobei die erste flüssige Zusammensetzung gegebenenfalls eine Lösung von PLGA in Aceton ist.

12. Verfahren nach Anspruch 10 oder 11, wobei die dritte flüssige Zusammensetzung polymere Nanopartikel umfasst.

13. Verfahren nach einem der Ansprüche 1 bis 9, wobei die erste flüssige Zusammensetzung mit der zweiten flüssigen Zusammensetzung unmischbar ist.

14. Verfahren nach Anspruch 13, wobei die erste flüssige Zusammensetzung ein organisches Lösungsmittel ausgewählt aus Ethylacetat und/oder Dichlormethan umfasst, und wobei die erste flüssige Zusammensetzung vorzugsweise eine Lösung von PLGA in Dichlormethan ist.

15. Verfahren nach Anspruch 13 oder 14, des Weiteren umfassend einen Schritt:
(g) Entfernen des organischen Lösungsmittels aus den dritten flüssigen Zusammensetzungen, um so polymere Mikropartikel zu erhalten, und wobei das organische Lösungsmittel vorzugsweise durch Lösungsmittelaustausch, Lösungsmittelextraktion und/oder Lösungsmittelverdampfung entfernt wird.

## Revendications

1. Procédé de préparation de microparticules ou nanoparticules polymères comprenant un agent biologiquement actif, le procédé comprenant les étapes suivantes :
(a) fournir un réacteur à impact de jet comprenant une chambre de réaction définie par une surface intérieure d'une paroi de chambre, la chambre de réaction ayant une forme générale sensiblement sphéroïdale, ladite chambre comprenant
- une première entrée de fluide et une deuxième entrée de fluide, les première et deuxième entrées de fluide étant disposées à des positions opposées sur un premier axe central de la chambre de réaction de manière à pointer l'une vers l'autre, et chacune des première et deuxième entrées de fluide comprenant une buse ; et
- une sortie de fluide disposée à une troisième position, ladite troisième position étant située sur un deuxième axe central de ladite chambre, le deuxième axe central étant perpendiculaire au premier axe central ;
(b) fournir un premier flux d'une première composition liquide, la première composition liquide comprenant une solution organique
- d'un polymère choisi parmi PLA, PGA, PLGA et des combinaisons de ceux-ci ;
et
- d'agent biologiquement actif ;
(c) fournir un deuxième flux d'une deuxième composition liquide, la deuxième composition liquide étant une solution aqueuse ;
(d) injecter simultanément le premier flux à un premier débit par la première entrée de fluide et le deuxième flux à un deuxième débit par la deuxième entrée de fluide dans la chambre de réaction du réacteur à impact de jet de manière à permettre à la première composition liquide et à la deuxième composition liquide de se mélanger et de former ainsi une troisième composition liquide ;
(e) permettre à la troisième composition liquide de s'écouler hors de la chambre de réaction par la sortie de fluide ; et
(f) collecter la troisième composition liquide.

2. Procédé selon la revendication 1, la distance entre la buse de la première entrée de fluide et la buse de la deuxième entrée de fluide étant inférieure ou égale au diamètre de la chambre de réaction le long du premier axe central.

3. Procédé selon la revendication 1 ou 2, les buses étant disposées de manière à diriger le premier flux et le deuxième flux le long du premier axe central vers le centre de la chambre de réaction et à permettre la collision du premier flux et du deuxième flux avec un angle d'environ 180°.

4. Procédé selon l'une quelconque des revendications précédentes, la chambre de réaction étant dépourvue d'autres entrées ou sorties.

5. Procédé selon l'une quelconque des revendications précédentes, la buse de la première entrée ayant un premier diamètre d'orifice et la buse de la deuxième entrée ayant un deuxième diamètre d'orifice, le premier diamètre d'orifice et/ou le deuxième diamètre d'orifice étant dans la gamme allant de 20 µm à 500 µm, et le premier diamètre d'orifice étant éventuellement supérieur au deuxième diamètre d'orifice, le rapport du premier diamètre d'orifice au deuxième diamètre d'orifice étant éventuellement de 1,2 à 5.

6. Procédé selon l'une quelconque des revendications précédentes, le polymère compris dans la première composition liquide comprenant du PLGA, et le PLGA ayant éventuellement un rapport lactide:glycolide de 75:25, un groupe terminal acide et un poids moléculaire moyen d'environ 20 kDa.

7. Procédé selon l'une quelconque des revendications précédentes, la première composition liquide ayant une viscosité ne dépassant pas 40 mPas*s.

8. Procédé selon l'une quelconque des revendications précédentes, la deuxième composition liquide comprenant un tensioactif, le tensioactif étant éventuellement un tensioactif non ionique choisi parmi les polysorbates tels que le polysorbate 20 et les poloxamères.

9. Procédé selon l'une quelconque des revendications précédentes, le deuxième débit étant supérieur au premier débit, et le rapport du premier débit au deuxième débit étant de préférence dans la gamme allant de 1,5:1 à 20:1, et en particulier de 2:1 à 10:1, et la somme du premier débit et du deuxième débit étant de préférence d'au moins 50 ml/min.

10. Procédé selon l'une quelconque des revendications précédentes, la première composition liquide étant miscible à la deuxième composition liquide.

11. Procédé selon la revendication 10, la première composition liquide comprenant un solvant organique choisi parmi l'acétone, le DMSO, le méthanol, l'acétonitrile ou toute combinaison de ceux-ci, et la première composition liquide étant éventuellement une solution de PLGA dans l'acétone.

12. Procédé selon la revendication 10 ou 11, la troisième composition liquide comprenant des nanoparticules polymères.

13. Procédé selon l'une quelconque des revendications 1 à 9, la première composition liquide étant non-miscible avec la deuxième composition liquide.

14. Procédé selon la revendication 13, la première composition liquide comprenant un solvant organique choisi parmi l'acétate d'éthyle et/ou le dichlorométhane, et la première composition liquide étant de préférence une solution de PLGA dans du dichlorométhane.

15. Procédé selon la revendication 13 ou 14, comprenant en outre l'étape suivante
(g) éliminer le solvant organique des troisièmes compositions liquides de manière à obtenir des microparticules polymères, et le solvant organique étant de préférence éliminé par échange de solvant, extraction de solvant et/ou évaporation de solvant.
